# EUROPÄISCHE PATENTSCHRIFT

(11) **EP 0 554 657 B1**
(45) Veröffentlichungstag und Bekanntmachung des Hinweises auf die Patenterteilung: **16.12.1998**
(21) Anmeldenummer: 93100040.0
(22) Anmeldetag: 05.01.1993
(51) Int. Cl.: G01N 33/53, G01N 33/564

(54) **Verfahren zur Bestimmung von Antigenen oder Antikörpern in Gegenwart eines Immunkomplexes**
Method for the determination of antigens or antibodies in the presence of an immune complex
Méthode pour la détermination d'antigènes ou d'anticorps dans la présence d'une complexe immunologique

(30) Priorität: 01.02.1992 DE 4202923
(43) Veröffentlichungstag der Anmeldung: 11.08.1993
(73) Patentinhaber: Dade Behring Marburg GmbH, 35001 Marburg (DE)
(72) Erfinder: Toth, Tibor, W-3550 Marburg (DE)

(56) Entgegenhaltungen:
- EP-A- 0 068 344
- US-A- 4 547 466

## Beschreibung

Die Erfindung betrifft ein immunchemisches Verfahren zum Nachweis oder zur Bestimmung eines Partners einer Antigen-Antikörper-Reaktion wobei zur Unterdrückung unspezifischer Reaktionen die immunchemische Reaktion in Gegenwart eines Immunkomplexes erfolgt, der keinen für einen der Partner spezifischen Antikörper oder Antigen enthält.

Bekanntlich verbinden sich spezifische Antikörper mit den entsprechenden Antigenen oder Haptenen. Von dieser Reaktion wird in vielen Immunoassay-Verfahren Gebrauch gemacht. Menschliche Seren können auf die Anwesenheit eines bestimmten Antigens unter Verwendung des entsprechenden Antikörpers beispielsweise mittels Nephelometrie, Turbidimetrie, ELISA oder der Latex-Agglutinationsreaktion untersucht werden. Diese und ähnliche Verfahren sind dem Fachmann wohl bekannt.

Es ist bekannt, daß diese Nachweisreaktionen durch bestimmte in dem zu untersuchenden Serum vorhandene Bestandteile gestört werden können. Insbesondere enthält das menschliche Serum das Protein C1q (eine Komplementkomponente) und Rheumafaktoren (RF). Von diesen Substanzen ist bekannt, daß sie gleichfalls an den Antikörper binden können. Die Konzentrationen der Rheumafaktoren und des C1q in menschlichen Seren variiert in sehr weiten Grenzen, weshalb es normalerweise erforderlich ist, die Seren vorher einer Behandlung zur Inaktivierung von C1q und/oder der Entfernung endogener Rheumafaktoren zu unterziehen. Andernfalls können die Ergebnisse und insbesondere die quantitativen Bestimmungen beträchtliche Fehler aufweisen.

Der Nachweis von Antigenen oder Antikörpern mit Hilfe der manuellen Latex-Agglutinationsreaktion hat den Vorteil, daß die Durchführung einfach ist und die Testergebnisse in sehr kurzer Zeit erhalten werden.

Nephelometrische und turbidimetrische Bestimmungsmethoden haben wegen ihrer hohen Präzision, Schnelligkeit und Automatisierbarkeit Zugang zu medizinischen Untersuchungs-Laboratorien gefunden.

Diese Methoden machen von der Eigenschaft von Antigenen und/oder Antikörpern Gebrauch, mit dem entsprechenden Partner in einer immunchemischen Reaktion Immunkomplexe zu bilden. Die nach Mischung beider Partner einsetzende Bildung von Antigen-/Antkörper-Komplexen und die damit einhergehende Veränderung der Zahl und Größe der Streuzentren kann dann z. B. photometrisch gemessen werden.

Es ist bekannt, die Empfindlichkeit serologischer oder immunologischer Bestimmungsverfahren durch die Verwendung von Indikator- oder Trägerteilchen zu erhöhen, die mit dem entsprechenden immunologischen Reagenz (einem Antikörper oder Antigen) beladen sind. Als Trägermaterial können zum Beispiel rote Blutkörperchen, Zellen einer Zellkultur oder Polymerpartikel verwendet werden. Meistens werden Latexpartikel mit einem Durchmesser von 0,02 bis 5 µm hierfür eingesetzt.

Ein solcher "partikelverstärkter" nephelometrischer oder turbidimetrischer Test kann Proteine bis zu Konzentrationen von etwa 5 ng/ml sicher nachweisen. Bei einem derartigen partikelverstärkten Test werden an partikuläre Polymere gebundene Antikörper bzw. Antigene ("Festphase") eingesetzt. Zur Bestimmung eines Antigens kommt ein festphasengebundener Antikörper zum Einsatz, zur Bestimmung eines Antikörpers ein festphasengebundenes Antigen. In beiden Fällen erfolgt durch die Immunreaktion eine Agglutination der Polymerpartikel. Daraus resultiert eine Größenzunahme der Agglutinate und damit verbunden eine Veränderung des Streulichtsignals bzw. der Trübung des Reaktionsansatzes.

Aus der EP 0 087 728 und EP 0 290 117 sind Latex-Agglutinationsverfahren unter Verwendung von Antiseren und/oder von Gammaglobulinen ohne Antikörperspezifität bezüglich eines an der Umsetzung beteiligten Reaktionspartners zur Verhinderung von unspezifischen Reaktionen bekannt.

Als solche Antiseren eignen sich tierische Gammaglobuline oder hitzeaggregierte humane Gammaglobuline, wie z. B. ein anti-Schaf-Erythrozyten-Serum von einem Säugetier und vorzugsweise ein anti-Schaf-Erythrozyten-Serum vom Kaninchen. Gammaglobuline im obengenannten Sinne sind auch Gammaglobulinfraktionen, die mittels bekannter Verfahren, beispielsweise Ammonsulfat-Fällung, Ionenaustauscher-Chromatographie oder durch Immunadsorptions-Chromatographie gewonnen werden können.

Nachteilig bei diesem Verfahren ist, daß die verwendeten Gammaglobulinlösungen gewöhnlich von denselben Tierspezies sein müssen, wie die an die Latex-Teilchen gebundenen spezifischen Nachweisantikörper gegen das zu bestimmende Antigen. So werden z. B. bei der Bestimmung von Myoglobin Antikörper gegen das Human-Myoglobin vom Kaninchen verwendet und die Absorptionslösung enthält ebenfalls Kaninchen-Gammaglobulin (immunisiert mit Schaf-Erythrozyten (Ambozeptor)). Diese Absorptionslösung ist nicht brauchbar bei einem Latex-Agglutinationstest zum Nachweis von CRP, bei dem die Antikörper z. B. gegen human-CRP vom Schaf sind, weil der Ambozeptor vom Kaninchen ist und eine spontane Agglutination der Latex-Teilchen verursacht.

Es ist möglich, wie in der EP 0 087 728 beschrieben, die Rheumafaktoren-Störung bei der Bestimmung von CRP mit anti-human IgG vom Schaf zu beseitigen. Wenn jedoch die festphasengebundenen Nachweisantikörper gegen human-CRP vom Schaf sind, dann reagiert in verdünnten Seren das im Serum vorhandene human-lgG mit dem anti-human IgG (Schaf) unter Agglutination, wenn das human-lgG etwa im Äquivalenzbereich (zu dem zugesetzten anti-human IgG) liegt.

In der EP-0 068 344 werden Verfahren zum Nachweis eines Antigens oder Antikörpers mittels ELISA offenbart, worin zwecks Elimination von Störeinflüssen durch bestimmte Serumfaktoren vor der Bestimmung bestimmte Substanzen, nämlich EDTA, Kaolin, ein Antigen-Antikörperkomplex, ein Ammoniumsalz, Zymosan oder anti-Clq zur Probe zugesetzt werden. Im Falle des Zusatzes eines Antigen-Antikörperkomplexes ist es gemäß der EP-0 068 344 aber zwingend erforderlich, eine bestimmte Inkubationszeit bei Raumtemperatur einzuhalten und die Probe vor der ELISA-Bestimmung einer Zentrifugation zur Abtrennung des Sediments zu unterwerfen.

Es wurde nun überraschenderweise gefunden, daß die vorstehend genannten Schwierigkeiten, die verursacht werden durch eine nicht-spezifische Agglutination, verhindert werden können, wenn die Agglutinationsreaktion in Gegenwart eines Immunkomplexes durchgeführt wird, dessen Bestandteile weder mit dem zu bestimmenden Antigen oder Antikörper noch mit dem an die Latexpartikel gebundenen Partner reagieren.

Gegenstand der Erfindung ist demnach ein Verfahren zum Nachweis und oder zur Bestimmung eines Partners (Analyt) einer immunologischen Reaktion in einer Probe biologischen Materials, wobei die spezifische immunchemische Reaktion in Gegenwart eines Immunkomplexes abläuft, der keine Antikörper/Antigen enthält, die für einen der Partner spezifisch sind.

Bevorzugt ist dabei ein Verfahren wie oben beschrieben, wobei der Immunkomplex der Probe vor der Zugabe des nachweisenden Partners der immunologischen Reaktion zugegeben wird.

Bevorzugterweise erfolgt die Zugabe des Immunkomplexes dabei in einem Zeitraum von weniger als 10 min vor der Zugabe des nachweisenden Partners. Der Immunkomplex kann auch mit dem nachweisenden Partner vorinkubiert oder gleichzeitig mit diesem zur Probe gegeben werden.

Bevorzugt ist femer ein Verfahren wie oben beschrieben, wobei der Immunkomplex Antikörper von immunisierten Tieren enthält, die gegen ein Antigen gerichtet sind, das nicht der Analyt ist. Das Antigen kann dabei humanen, tierischen oder pflanzlichen Ursprungs sein.

Bevorzugterweise ist das Verhältnis von Antikörper : Antigen im Immunkomplex 1 : 0,5 - 1 : 5, ganz bevorzugterweise etwa 1 : 1.

Im folgenden werden Verfahren zur Herstellung von für die erfindungsgemäßen Verfahren geeigneten Immunkomplexen offenbart.

Da bei der partikel-verstärkten Agglutinationsmethode meist ein Fällungsmittel wie Polyäthylenglycol (PEG) verwendet wird, dürfen die Komplexe nicht durch das Fällungsmittel ausgefällt werden. Außerdem müssen sie über einen längeren Zeitraum, das heißt in der Regel mindestes 3 Monate, bevorzugterweise mindestens 12 Monate, lagerstabil sein.

Immunkomplexe im Sinne der Erfindung sind alle Immunkomplexe, die nach bekannten Verfahren, d. h. durch Vermischen von Lösungen des Antigens mit dem Antikörper er-halten werden können. Anstelle des kompletten Gamma-globulins können auch jeweils ihre F_{c}-Fragmente verwendet werden. Die verwendeten Antiköper können polyklonal (Antiseren) oder auch monoklonal sein. Als Beispiele wer-den genannt Komplexe aus anti-human-lgG vom Schaf/lgG, anti-human-IgM vom Kaninchen/lgM, Antikörper gegen Kaninchen-Gammaglobulin von der Ziege/ Kaninchen-Gammaglobulin, Antikörper gegen Rinderkasein vom Kaninchen/Kasein, Antikörper gegen Soja-Protein vom Kaninchen/Soja-Protein.

Besonders geeignet sind Komplexe aus Antikörpern gegen Rinder-Gammaglobulin vom Kaninchen/Rinder-Gammaglobulin, anti-human-IgG vom Schaf/IgG und anti- human-IgG-F_{c} vom Kaninchen/IgG-F_{c}. Die Herstellung erfolgt in einer wäßrigen Lösung, vorzugsweise in Gegenwart eines polaren, mit Wasser gut mischbaren Lösungsmittels wie Dimethylsulfoxid oder Dimethylformamid.

Bevorzugt ist die Herstellung der Komplexe in Gegenwart eines zyklischen Amids, besonders geeignet ist Pyrrolidon ( -Amino-Butyrolactam). Bevorzugterweise wird das Amid in einer Konzentration von 1 bis 50 Volumenprozent, besonders bevorzugterweise 5 bis 30 Volumenprozent verwendet. Die so hergestellten Immunkomplexe sind in wässrigen Lösungen mindestens 3, bevorzugterweise mindestens 12 Monate lagerstabil.

Das erfindungsgemäße Verfahren ist auf alle Reaktionen zum Nachweis von immunologisch aktiven Substanzen, die im Blut (Serum oder Plasma) von Säugetieren, insbesondere , vom Menschen und in Körperflüssigkeiten (Liquor), enthalten sind, anwendbar. Beispiele solcher immunologisch aktiver Substanzen sind Serumproteine.

Ein wesentliches Merkmal der Erfindung ist seine weitreichende Anwendbarkeit.

Als Latex-Teilchen, die mit den immunologisch aktiven Substanzen beladen (sensibilisiert) werden, kommen alle Latices in Frage, die für den Latex-Agglutinationstest geeignet sind. Als Beispiele seien Homo- und Copolymere von Styrol genannt. Bevorzugt werden Latices mit einer Teilchengröße von 0,05 bis 0,6 µm. Verfahren zur Herstellung solcher Partikel sind dem Fachmann bekannt.

Ein Partner einer immunologischen Reaktion kann adsorptiv oder durch kovalente Bindung auf die Partikel aufgebracht und auf eine solche Weise "beladen" werden. Die Kupplung der Partikel mit einem Antigen oder Antikörper kann nach dem Fachmann bekannten Methoden durchgeführt werden. Als Antikörper können dabei sowohl mono- als auch polyklonale Antikörper verwendet werden.

Die Sensibilisierung der vorstehend genannten Latex-Teilchen mit den Antigenen oder Antikörpern kann nach einer bekannten Methode durchgeführt werden. Bevorzugt wird das Latex mit Antikörpern gegen Serumproteine wie Myoglobin, Beta 2-Mikroglobulin, CRP oder Immunglobulin E, humanen Hormonen wie Human-Choriogonadotropin, Enzymen wie Pankreas-Lipase oder tierische Hormonen wie Pregnant mare serum gonadotropin beladen. Es kann dann wie folgt sensibilisiert werden: Aus einem Antiserum werden die Gammaglobuline in üblicher Weise, beispielsweise mit Ammoniumsulfat, ausgefällt, die Gammaglobulinfraktion dialysiert und auf 30 - 50 g/l konzentriert. Es können auch reine Antikörperlösungen immunadsorptiv gewonnen und auf 2 - 10 g/l konzentriert werden. Eine Suspension der Latex-Teilchen mit einer Konzentration von etwa 20 - 200 g/l, bevorzugterweise etwa 100 g/l wird mit der Antikörper-Lösung versetzt und 0,5 - 5 Stunden bei einer Temperatur zwischen 20 und 60 °C inkubiert. Der nicht an Latex-Teilchen gebundene Anteil der Antikörper kann durch Zentrifugation und Resuspension des Feststoffes entfernt werden. Zum Gebrauch kann das Reagenz in einer Pufferlösung, vorzugsweise Glycin-NaCl-Puffer von pH 7 - 8,5, resuspendiert werden, die gegebenenfalls mit einem Protein, beispielsweise mit Rinder- oder Human-Albumin, versetzt werden kann.

Weitere Methoden sind dem Fachmann bekannt.

Die Auswertung der Testergebnisse kann visuell erfolgen oder mit einem Gerät z. B. nephelometrisch, turbidimetrisch oder auch mit Hilfe von dem Fachmann an sich bekannten Partikelzählverfahren vorgenommen werden. Erfolgt die Auswertung an einem Automaten, so wird der Immunkomplex direkt zu der zu untersuchenden Probe dosiert. Die Inkubation der Probe mit dem Immunkomplex kann aber auch in einem separaten Schritt durchgeführt werden.

Folgende Beispiele erläutern die Erfindung:

### Beispiel 1:

### Herstellung des Immunkomplexes

1 a) 450 ml (20 g/l) human-Gammaglobulin in isotonischer Kochsalzlösung wurden mit 250 ml Antiserum gegen human-IgG vom Schaf, die mit 250 ml isotonischem Kochsalz verdünnt waren, unter intensivem Rühren versetzt.
   Die Lösung wurde danach ca. 10 Stunden bei +56 °C inkubiert und nach Abkühlen mit 50 ml Pyrrolidon versetzt. Zur Konservierung können 2 g/l Benzamidiniumchlorid und 1 g/l Natriumazid zugesetzt werden.
1 b) 450 ml (20 g/l) human-Gammaglobulin in isotonischer Kochsalzlösung wurden mit 400 ml Antiserum gegen human-Gammaglobulin vom Kaninchen, die mit 100 ml isotonischer Kochsalzlösung verdünnt waren, unter intensivem Rühren zugegeben. Die Lösung wurde 30 min bei +56 °C inkubiert und mit 50 ml Pyrrolidon, 2 g/l Benzamidiniumchlorid und 1 g/l Natriumazid versetzt.

Nach dem Stand der Technik hergestelltes Latex-Reagenz, welches als spezifische Antikörper Antikörper gegen human-CRP vom Schaf an Latex-Teilchen gebunden enthält, wurde erfindungsgemäß in folgendem Test eingesetzt. Die Empfindlichkeit des Reagenzes wurde an einem CRP-Standard auf ca. 6 mg/l eingestellt und dieser Test wie folgt ausgeführt:
1 Tropfen unverdünntes, zu prüfendes Human-Serum (50 µl) wurde auf ein Feld einer Testplatte gegeben, 50 µl Immunkomplex-Lösung (hergestellt nach 1 a) aus Antiserum gegen human-IgG/human-IgG zugegeben und ein Tropfen (40 µl) Latex-CRP-Reagenz zugesetzt. Nach Durchmischen mittels eines Rührstäbchens wurde die Testplatte rotierend bewegt und nach 2 min auf Agglutination geprüft.

Die folgende Tabelle veranschaulicht die Zuverlässigkeit des Verfahrens bei der Bestimmung von CRP im Serum:

| Serum Nr. | Konzentration RF IU/ml | CRP* mg/l | Latex-Test gemäß Stand d. Technik | Erfindung |
|---|---|---|---|---|
| 1 | 708 | neg | + | - |
| 2 | 529 | 3,8 | + | - |
| 3 | 512 | 15,7 | + | + |
| 4 | 555 | 3,8 | + | - |
| 5 | 525 | neg | + | - |
| 6 | 425 | 4,7 | + | - |
| 7 | 612 | 4,1 | + | - |
| 8 | 831 | 4,4 | + | - |
| 9 | 2 300 | 20 | + | + |
| 10 | < 20 | 10,6 | + | + |
| 11 | < 20 | 14,2 | + | + |
| 12 | < 20 | 70 | + | + |
| 13 | < 20 | neg | - | - |
| 14 | < 20 | neg | - | - |

| | | | | |
|---|---|---|---|---|
| *bestimmt nach der Radialen Immundiffusion | | | | |

Die klinisch interessierende Grenze liegt bei 6 mg/l. Wie die Tabelle zeigt, wurden Latex-Teste nach Stand der Technik durch Rheumafaktoren gestört (vgl. Interference by Rheumatoid Factor with the Detection of C-Reactive Protein by the Latex Agglutination Method. Deyo et al., J. of Rheumatology 7, 3 (1980) 279), in dem erfindungsgemäßen jedoch alle Proben richtig wiedergefunden.

### Beispiel 2:

### 1) Herstellung des Immunkomplexes

15 ml (16 g/l) human-Gammaglobulin in 50 ml Glycin-Kochsalzlösung pH 8,2, wurden mit 250 ml Antiserum gegen human-IgG vom Kaninchen unter intensivem Rühren versetzt. Die Lösung wurde danach ca. 5 Std. bei +56 °C inkubiert und nach Abkühlen mit 5 ml Pyrrolidon versetzt.
2) Latex-HCG-Reagenz wurde für die nephelometrische Messung auf ca. 0,05 g/l Feststoff verdünnt und mit Ultraschall behandelt. Es wurde ein Standard-Serum mit 12 500 mIU/ml eingesetzt; die Standardreihe am Gerät automatisch 1:20 bis 1:1280 verdünnt, d. h. es wurden Konzentrationen von 625 bis 9,8 mIU/ml erhalten.

Die zu bestimmenden Seren wurden mit einer Phosphat-Kochsalz-Pufferlösung verdünnt. Zur Messung wurden 50 µl Patientenserum und 10 µl des Immunkomplex-Reagenzes (hergestellt aus Antiserum gegen human-IgG (vom Kaninchen)/IgG eingesetzt. Die Messung erfolgte nach 12 min bei Raumtemperatur an einem Nephelometer (z. B. Behringwerke AG). Die Referenzkurve für die Messung des Standard-Serums wurde aufgezeichnet und die Patientenseren daran ausgewertet.

| Serum Nr. | HCG-Gehalt bei Serumverdünnung (mIU/ml) mit Immunkomplex-Reagenz ohne Ik-Reagenz | | | | |
|---|---|---|---|---|---|
| | RF-Gehalt | 1:1 | 1:20 | 1:100 | 1:20 |
| | IU/ml | | | | |
| 1 | 380 | < 9,8 | < 195 | < 977 | 737 |
| 2 | 420 | < 9,8 | < 195 | < 977 | 608 |
| 3 | 486 | < 9,8 | < 195 | < 977 | 1 020 |
| 4 | 580 | < 9,8 | < 195 | < 977 | 351 |
| 5 | 604 | < 9,8 | < 195 | < 977 | 551 |
| 6 | neg. | - | 7 260 | 8 200 | 7 920 |
| 7 | neg. | - | 16 900 | 18 020 | 17 300 |
| 8 | 280 | 18 | < 195 | - | 326 |
| 9 | 496 | 80 | < 195 | - | 512 |
| 10 | < 18 | < 9,8 | < 195 | < 977 | < 195 |
| 11 | < 18 | < 9,8 | < 195 | < 977 | < 195 |

Die klinisch interessierende Grenze liegt bei ca. 10 mIU/ml. Bei den Seren Nr. 6 und Nr. 7 handelt es sich um Seren von schwangeren Frauen. Wie aus der Tabelle zu entnehmen ist, zeigen die Seren 1 bis 5, 8 und 9 wegen der RF-Störung, ohne Zusatz von Immunkomplex, einen falschen, "zu hohen" Wert an HCG, an.

### Beispiel 3

1. 63 ml (14 g/l) human-IgG-F_{c} in isotonischer Kochsalzlösung wurden mit 100 ml anti-human-IgG-Gammaglobulin-Fraktion vom Kaninchen (etwa 50 g/l Protein) unter intensivem Rühren versetzt. Die Lösung wurde 2 Stunden bei + 56 °C inkubiert und nach Abkühlung auf Raumtemperatur mit 7 ml Pyrrolidon versetzt.
2. Latex-IgM-Reagenz wurde für eine nephelometrische Messung auf etwa 0,06 g/l Feststoff verdünnt und mit Ultraschall behandelt. Es wurde ein Standardserum mit 960 mg/l eingesetzt; eine Standardreihe wurde im Gerät automatisch 1 : 100 bis 1: 6.400 verdünnt, d. h. es wurden Konzentrationen von 10 bis 0,16 mg/l erhalten. Die zu bestimmenden Seren wurden mit einer Phosphat-Kochsalz-Pufferlösung verdünnt. Zur Messung wurden 50 µl Patientenserum mit 10 µl eines Immunkomplex-Reagenzes versetzt. Jede Ablesung erfolgte nach 12 Minuten.

| Serum | RF-Gehalt | IgM-Gehalt (mg/l) | | |
|---|---|---|---|---|
| | IU/ml | ohne IK | mit IK-Reagenz | RID* |
| 1 | 490 | > 4 000 | 1 070 | 971 |
| 2 | 596 | 3 800 | 1 660 | 1 709 |
| 3 | 660 | 3 470 | 1 570 | 1 576 |
| 4 | neg. | 1 130 | 1 260 | 1 152 |
| 5 | neg. | 499 | 506 | 535 |

| | | | | |
|---|---|---|---|---|
| * IgM-Konzentration mit der Methode der radialen Immunodiffusion | | | | |

Die Rheumafaktoren positiven Seren 1 - 3 zeigen ohne Immunkomplex-Reagenz einen falsch "zu hohen" Wert von IgM an.

## Patentansprüche

1. Agglutinationsverfahren zum Nachweis oder zur Bestimmung eines Partners (Analyt) einer immunologischen Reaktion in einer Probe biologischen Materials, dadurch gekennzeichnet, daß die spezifische immunchemische Reaktion in Gegenwart eines Immunkomplexes abläuft, der keine Antikörper/Antigen enthält, die für einen der Partner spezifisch sind.

2. Verfahren nach Anspruch 1, dadurch gekennzeichnet, daß der Immunkomplex der Probe vor der Zugabe des nachweisenden Partners der immunologischen Reaktion zugegeben wird.

3. Verfahren nach Anspruch 1, dadurch gekennzeichnet, daß der Immunkomplex Antikörper von immunisierten Tieren enthält, die gegen ein Antigen gerichtet sind, das nicht der Analyt ist.

4. Verfahren nach Anspruch 1, dadurch gekennzeichnet, daß das Antigen ein Protein humanen, tierischen oder pflanzlichen Ursprungs ist.

5. Verfahren nach Anspruch 1, dadurch gekennzeichnet, daß das Verhältnis im Immunkomplex von Antikörper: Antigen = 1 : 0,5 - 1 5 beträgt.

6. Verfahren nach Anspruch 1, dadurch gekennzeichnet, daß der Immunkomplex in einer wäßrigen Lösung hergestellt wird.

7. Verfahren nach Anspruch 1, dadurch gekennzeichnet, daß dem Immunkomplex bis zu 50 % eines cyclischen Amids zugesetzt wurde.

## Claims

1. An agglutination method for the detection or the determination of one partner (analyte) of an immunological reaction in a sample of biological material, wherein the specific immunochemical reaction takes place in the presence of an immune complex which does not contain any antibodies/antigens that are specific for one of the partners.

2. The method as claimed in claim 1, wherein the immune complex is added to the sample before the addition of the detecting partner of the immunological reaction.

3. The method as claimed in claim 1, wherein the immune complex contains antibodies from immunized animals that are directed against an antigen which is not the analyte.

4. The method as claimed in claim 1, wherein the antigen is a protein of human, animal or vegetable origin.

5. The method as claimed in claim 1, wherein the antibody : antigen ratio in the immune complex is 1 : 0.5 - 1 : 5.

6. The method as claimed in claim 1, wherein the immune complex is prepared in an aqueous solution.

7. The method as claimed in claim 1, wherein up to 50% of a cyclic amide was added to the immune complex.

## Revendications

1. Procédé d'agglutination pour la caractérisation ou pour la détermination d'un partenaire (analyte) d'une réaction immunologique dans un échantillon de matériaux biologiques, caractérisé en ce que la réaction immunochimique spécifique est effectuée en présence d'un complexe immun qui ne contient ni anticorps, ni antigène, qui sont spécifiques d'un des partenaires.

2. Procédé selon la revendication 1, caractérisé en ce que le complexe immun de l'échantillon est ajouté avant l'addition du partenaire de la réaction immunologique à caractériser.

3. Procédé selon la revendication 1, caractérisé en ce que le complexe immun contient des anticorps d'animaux immunisés, qui sont dirigés contre un antigène, qui n'est pas l'analyte.

4. Procédé selon la revendication 1, caractérisé en ce que l'antigène est une protéine d'origine humaine, animale ou végétale.

5. Procédé selon la revendication 1, caractérisé en ce que, dans le complexe immun le rapport entre l'anticorps et l'antigène est compris entre 1:0,5 et 1:5.

6. Procédé selon la revendication 1, caractérisé en ce que le complexe immun est préparé dans une solution aqueuse.

7. Procédé selon la revendication 1, caractérisé en ce qu'on ajoute au complexe immun une quantité allant jusqu'à 50% d'un amide cyclique.
